# FASCICULE DE BREVET EUROPEEN

(11) **EP 0 599 971 B1**
(45) Date de publication et mention de la délivrance du brevet: **02.05.2003**
(21) Numéro de dépôt: 92918265.7
(22) Date de dépôt: 19.08.1992
(51) Int. Cl.: C12N 15/81, C12N 1/19, C12P 21/02

(54) **PROMOTEUR DE LEVURE ET SON UTILISATION**
HEFEPROMOTOR UND SEINE VERWENDUNG
YEAST PROMOTER AND USE THEREOF

(30) Priorité: 21.08.1991 FR 9110476
(43) Date de publication de la demande: 08.06.1994
(73) Titulaire: Aventis Pharma S.A., 92160 Antony (FR)
(72) Inventeur: FALCONE, Claudio, I-00197 Rome (IT); FLEER, Reinhard, F-91440 Bures-sur-Yvette (FR); SALIOLA, Michele, I-00172 Rome (IT)
(86) Numéro de dépôt international: FR9200803
(87) Numéro de publication internationale: WO93004176

(56) Documents cités:
- FR-A- 2 635 115
- YEAST vol. 7, no. 4, Juin 1991, CHICESTER, UK pages 391 - 400 M. SALIOLA ET AL. 'Two genes encoding putative mitochondrila alcohol dehydrogenases are present in the yeast Kluyveromyces lactis'
- BIOTECHNOLOGY vol. 8, no. 2, Février 1990, NEW YORK US pages 135 - 139 J.A. VAN DEN BERG ET AL. 'Kluyveromyces as a host for heterologous gene expression: expression and secretion of prochymosin'

## Description

La présente invention concerne le domaine de la biologie moléculaire. Plus particulièrement, elle concerne une nouvelle séquence d'ADN présentant une activité de promoteur transcriptionnel, des vecteurs d'expression contenant cette séquence, et son utilisation pour la production de protéines recombinantes, et par exemple de protéines hétérologues. L'invention concerne aussi les cellules recombinées contenant cette séquence d'ADN.

Les progrès accomplis dans le domaine de la biologie moléculaire ont permis de modifier des microorganismes pour leur faire produire des protéines hétérologues. En particulier, de nombreuses études génétiques ont porté sur la bactérie E.coli. Toutefois, l'application industrielle de ces nouveaux modes de production est encore limitée, en particulier par les problèmes d'efficacité d'expression des gènes dans ces microorganismes recombinés. Aussi, dans le but d'augmenter les performances de ces systèmes de production, des recherches ont été effectuées afin d'isoler des promoteurs forts, permettant d'obtenir des niveaux élevés d'expression de protéines hétérologues. Chez E.coli, on peut citer en particulier les promoteurs des opérons tryptophane et lactose.

Plus récemment, chez la levure S.cerevisiae, des études ont porté sur des promoteurs dérivés de gènes impliqués dans la glycolyse. On peut citer notamment les travaux sur le promoteur du gène de la 3-phosphoglycérate kinase PGK (Dobson et al., Nucleic Acid Res. 10, 1982, 2625 ; Hitzeman et al., Nucleic Acid Research 1982, 7791), sur celui du gène de la glyceraldéhyde-3-phosphate deshydrogénase GAPDH (Holland et al., J. Biol. Chem. 254, 1979, 9839 ; Musti et al., Gene 25, 1983, 133), sur celui du gène de l'alcool deshydrogénase 1 ADH1 (Bennentzen et al., J. Biol. Chem. 257, 1982, 3018 ; Denis et al., J.Biol.Chem. 25, 1983, 1165), ou sur celui du gène de l'enolase 1 ENO1 (Uemura et al., Gene 45, 1986, 65).

Récemment, des outils génétiques ont été développés afin de se servir de la levure Kluyveromyces comme cellule hôte pour la production de protéines recombinantes. La mise en évidence d'un plasmide de type 2-micron originaire de K. drosophilarum (plasmide pKD1 - EP 241 435) a permis d'établir un système hôte/vecteur très efficace pour la production de protéines recombinantes (EP 361 991). Cependant, les promoteurs utilisés dans ce système n'ont jamais été optimisés. En particulier, il s'agit essentiellement de promoteurs hétérologues, c'est-à-dire provenant d'autres microorganismes, tel que notamment S.cerevisiae. Cette situation peut engendrer différents inconvénients, et notamment limiter l'activité du promoteur à cause de l'absence de certains éléments de la machinerie transcriptionnelle (par exemple de trans-activateurs), présenter une certaine toxicité pour la cellule hôte due à une absence de régulation, ou affecter la stabilité du vecteur.

Dans ces conditions, le manque de promoteurs homologues forts chez Kluyveromyces constitue un facteur limitant dans l'exploitation industrielle de ce système d'expression.

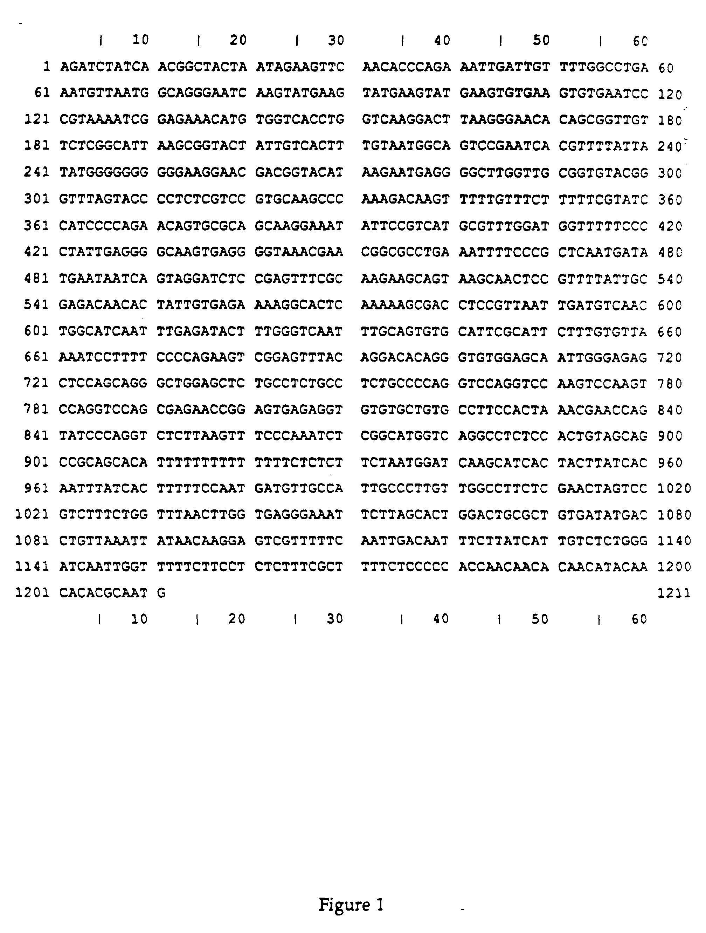

La Demanderesse a maintenant identifié, cloné et séquencé une région du génome de Kluyveromyces lactis présentant une activité de promoteur transcriptionnel (voir figure 1). Plus précisément, cette région correspond au promoteur du gène ADH4 de K. lactis (KlADH4) codant pour l'alcool déshydrogénase IV. Le gène KlADH4 a été identifié et cloné à partir d'une banque génomique de K.lactis (Saliola et al. yeast 7: 391-400 (1991) mais la région promotrice de ce gène n'a pas été identifiée et ses propriétés n'ont jamais été mises en évidence. La région, identifiée par la demanderesse ou des dérivés ou fragments de cette région, peut être utilisée de manière très performante pour la production de protéines recombinantes chez les levures du genre Kluyveromyces. Il est entendu que cette séquence peut également être utilisée dans d'autres organismes hôtes.

Par ailleurs, l'analyse de la région du génome de Kluyveromyces obtenue a permis de mettre en évidence une activité promotrice bidirectionnelle. Cette observation indique que le brin complémentaire de la région présentée sur la figure 1 possède également une activité promotrice agissant dans l'autre orientation.

De plus, un autre avantage de l'activité promotrice obtenue réside dans son caractère régulable. De ce fait, selon les conditions d'utilisation (milieu, souche), il est possible de contrôler l'activité du promoteur, et donc de déclencher ou de réprimer l'expression d'un gène recombiné.

Un autre avantage de la région promotrice obtenue réside dans l'absence de répréssion par le glucose. Ce résultat est surprenant puisqu'il s'agit du premier exemple de promoteur dérivé d'un gène ADH induit par l'éthanol et non réprimé par le glucose. En effet, dans le cas de S.cerevisiae, le gène ADH2 est exprimé sur différentes sources de carbone non fermentescibles (glycérol, éthanol, lactate, pyruvate), mais, contrairement à KlADH4, l'expression de ce gène est très fortement réprimée lorsque du glucose est ajouté au milieu (Denis et al., J. Mol. Biol. 148 (1981) 355). De même, l'expression du gène alcA qui code pour une alcool deshydrogénase I chez Aspergillus nidulans est induite par l'éthanol, mais sensible à la répression par le glucose (Falenbok, J.Biotechnol. 17 (1991) 11). Berg, J. Biotechnolog 8 (1990) 2 a trait au sous-clonage de fragments de promoteurs du Lac 4 gène de K.lactis. Des signaux permettant la sécrétion du produit d'expression sont aussi divulgués dans ce document.

Un objet de la présente invention réside donc dans une séquence d'ADN constituée de tout ou partie de la séquence présentée à la figure 1 ou de son brin complémentaire, ou d'un dérivé de celles-ci, et possédant une activité de promoteur transcriptionnel.

Au sens de la présente invention, on entend par dérivé, toute séquence obtenue à partir de la séquence donnée dans la figure 1 par modifications structurales (mutations, délétions, substitutions, additions, fragmentations ..) conservant une activité de promoteur. En particulier, les mutations peuvent porter sur un ou plusieurs nucléotides, et les additions et/ou substitutions peuvent porter sur des éléments de régulation, ou des régions activatrices telles que les "UAS".

Lorsqu'un dérivé est réalisé, son activité de promoteur transcriptionnel peut être mise en évidence de plusieurs façons, et en particulier en plaçant sous le contrôle de la séquence étudiée, un gène de résistance ou un marqueur de complémentation. Toute autre technique connue de l'homme de l'art peut bien évidemment être utilisée à cet effet.

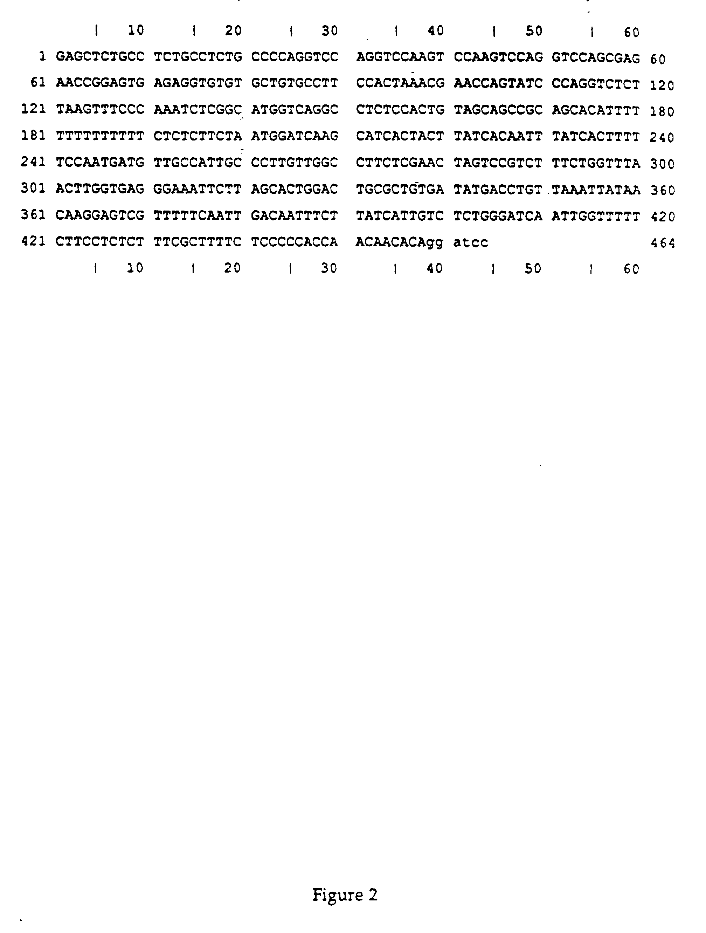

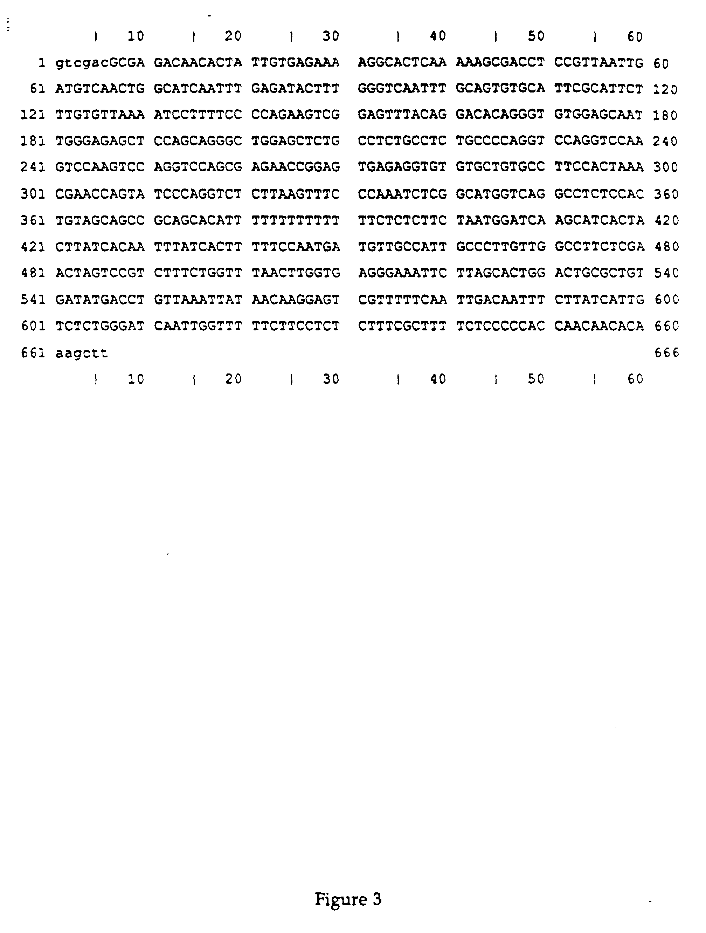

Comme exemples de dérivés selon l'invention, on peut citer plus particulièrement un promoteur comprenant le fragment SacI-BamHI de 500 pb environ dont la séquence est présentée sur la figure 2 ou un fragment SalI-HindIII de 700 pb environ dont la séquence est présentée sur la figure 3, ou le fragment BglII-SacI de 700 pb environ correspondant au fragment compris entre les nucléotides 1 et 723 du brin complémentaire de la séquence présentée sur la figure 1.

La construction de ces promoteurs est décrite en détail dans les exemples.

La séquence présentée sur la figure 1 a été obtenue à partir d'un fragment BamHI de 8 kb environ, obtenu par criblage d'une banque d'ADN génomique total de Kluyveromyces lactis au moyen d'une sonde hétérologue provenant du gène de structure ADH2 de S.cerevisiae. La Demanderesse a en effet montré qu'il est possible de cloner une région promotrice chez Kluyveromyces, par hybridation à partir de sondes hétérologues correspondant à un gène de S.cerevisiae. Les détails du clonage de la séquence sont donnés dans les exemples. Les dérivés selon l'invention peuvent ensuite être préparés à partir de ces séquences, comme indiqué dans les exemples.

Un autre objet de l'invention concerne un ADN recombinant comprenant une séquence d'ADN telle que définie ci-dessus et un ou plusieurs gènes de structure à l'exception du gène KlADH4 de K.lactis.

Cet ADN recombinant peut contenir par exemple la séquence promotrice présentée à la figure 1 ou un dérivé de celle-ci, dans laquelle est inséré un site de restriction, facilitant l'utilisation de cette séquence comme promoteur "portable".

Préférentiellement, cet ADN recombinant contient un ou plusieurs gènes de structure. En particulier, il peut s'agir de gènes codant pour des protéines d'intérêt pharmaceutique ou agroalimentaire. A titre d'exemple, on peut citer les enzymes (tels que notamment la superoxide dismutase, la catalase, les amylases, les lipases, les amidases, la chymosine etc.), les dérivés sanguins (tels que la sérum-albumine, l'alpha- ou la béta-globine, le facteur VIII, le facteur IX, le facteur de von Willebrand, la fibronectine, l'alpha-1 antitrypsine etc.), l'insuline et ses variants, les lymphokines (telles que les interleukines, les interférons, les facteurs de stimulation des colonies [G-CSF, GM-CSF, M-CSF...], le TNF, le TRF etc.), les facteurs de croissance (tels que l'hormone de croissance, l'érythropoiétine, le FGF, l'EGF, le PDGF, le TGF etc.), les apolipoprotéines, des polypeptides antigéniques pour la réalisation de vaccins (hépatite, cytomégalovirus, Eppstein-Barr, herpes etc.), ou encore des fusions de polypeptides telles que notamment des fusions comportant une partie active fusionnée à une partie stabilisatrice (par exemple des fusions entre l'albumine ou des fragments d'albumine et le récepteur ou une partie d'un récepteur de virus [CD4, etc.]).

Encore plus préférentiellement, l'ADN recombinant contient également des signaux permettant la sécrétion du produit d'expression du ou desdits gènes de structure. Ces signaux peuvent correspondre aux signaux naturels de sécrétion de la protéine considérée, mais ils peuvent également être d'une origine différente. En particulier des signaux de sécrétion dérivés de gènes de levure peuvent être utilisés, tels que ceux des gènes de la toxine killer (Stark et Boyd, EMBO J. 5 (1986) 1995) ou de la phéromone alpha (Kurjan et Herskowitz, Cell 30 (1982) 933 ; Brake et al., Yeast 4 (1988) S436).

Dans un mode de réalisation particulier de l'invention, l'ADN recombinant fait partie d'un plasmide d'expression, qui peut être à réplication autonome ou intégratif.

En particulier, des vecteurs à réplication autonome peuvent être obtenus en utilisant des séquences à réplication autonomes chez l'hôte choisi. Notamment, chez la levure, il peut s'agir d'origines de réplication dérivées de plasmides (pKD1, 2µ, etc), ou bien de séquences chromosomiques (ARS).

Les vecteurs intégratifs peuvent être obtenus notamment en utilisant des séquences homologues à certaines régions du génome de l'hôte, permettant, par recombinaison homologue, l'intégration du vecteur.

Un autre objet de l'invention concerne les cellules recombinées contenant une séquence d'ADN telle que définie ci-avant.

Avantageusement, les cellules sont choisies parmi les levures, et encore plus préférentiellement, parmi les levures du genre Kluyveromyces. Il est entendu cependant que l'invention couvre toutes les cellules recombinées dans lesquelles les régions promotrices de l'invention sont actives.

Ces cellules peuvent être obtenues par toute méthode permettant d'introduire un ADN étranger dans une cellule. Il peut s'agir notamment de transformation, électroporation, ou toute autre technique connue de l'homme de l'art.

Un autre objet de l'invention concerne l'utilisation d'une séquence telle que précédemment définie pour l'expression de gènes recombinés.

Comme l'illustrent les exemples, les séquences d'ADN selon l'invention permettent en effet une production à des niveaux élevés de protéines recombinantes.

Par ailleurs, l'activité promotrice bidirectionnelle des séquences de l'invention permet une utilisation particulièrement avantageuse. Notamment, il est possible d'utiliser ces séquences pour l'expression simultanée de plusieurs gènes recombinés dans les deux orientations opposées.

Avantageusement, l'invention concerne l'utilisation d'une séquence telle que précédemment définie pour l'expression simultanée de deux gènes recombinés insérés de part et d'autre du promoteur, dans les deux orientations opposées.

Avantageusement, les séquences de l'invention peuvent être utilisées pour l'expression de gènes codant pour des protéines d'intérêt pharmaceutique ou agroalimentaire. A titre d'exemple, on peut citer les protéines énumérées précédemment.

La présente invention permet également la réalisation d'un procédé de production de protéines recombinantes, selon lequel on cultive une cellule recombinée telle que définie ci-avant et on récupère la protéine produite. A titre d'exemple de protéine, on peut citer les protéines énumérées précédemment.

Préférentiellement, le procédé de l'invention est applicable à la production de sérum-albumine humaine, ou un de ses variants moléculaires. On entend par variant moléculaire de l'albumine, les variants naturels résultant du polymorphisme de l'albumine, les formes tronquées, ou toute protéine hybride à base d'albumine.

Par ailleurs, un aspect particulièrement avantageux de l'invention réside dans la possibilité de réguler l'activité des promoteurs. La demanderesse a en effet montré que l'activité promotrice était spécifiquement induite par l'éthanol. A cet effet, l'éthanol inducteur peut soit être ajouté directement au milieu de culture, soit être produit intracellulairement par la souche hôte, selon ses capacités de fermentation, à partir de la source de carbone introduite dans le milieu. La régulation peut donc être obtenue dans plusieurs conditions :
- soit en cultivant la cellule recombinée dans un milieu contenant une source carbonée différente de l'éthanol et non transformable en éthanol par la cellule. Dans ce cas, l'activité du promoteur est induite par addition d'éthanol dans le milieu. Par exemple, dans le cas de K.lactis 2359/152 et K.lactis MW98-8C, le promoteur KlADH4 est inactif lorsque les cellules sont cultivées sur un milieu contenant du glycérol, mais il est induit après addition d'éthanol.
- soit en cultivant, dans un milieu contenant une source carbonée fermentescible (par exemple le glucose), une cellule recombinée présentant une déficience au niveau de son métabolisme fermentatif, et de ce fait, incapable de produire de l'éthanol à partir de cette source carbonée. Dans ce cas, l'activité du promoteur est induite soit par addition d'éthanol dans le milieu, soit par addition d'un autre sucre fermentescible intervenant dans une étape en aval de celle déficiente, et capable d'être métabolisé en éthanol en dépit de ladite déficience. A titre d'exemple, on peut utiliser une souche présentant une mutation rag2, et de ce fait ne possédant pas l'activité phosphoglucose isomérase. Dans ce cas, le promoteur KlADH4 est inactif sur milieu glucose, et l'activité du promoteur peut être induite par addition d'éthanol ou de fluctose.

D'autres avantages de la présente invention apparaîtront à la lecture des exemples qui suivent, qui doivent être considérés comme illustratifs et non limitatifs.

### LEGENDE DES FIGURES

- Figure 1 :: Séquence nucléotidique du fragment de 1,2 kb correspondant au promoteur KlADH4 de K.lactis.
- Figure 2 :: Séquence nucléotidique du fragment SacI-BamHI de 0,5 kb environ, correspondant au promoteur KlADH4 tronqué dans le vecteur pP4-15Kan.
- Figure 3 :: Séquence nucléotidique du fragment SalI-HindIII de 0,7 kb environ, correspondant au promoteur KlADH4 tronqué dans les vecteurs pYG129 et pYG130.
- Figure 4 :: Représentation schématique du plasmide p6-4-98 et construction des plasmides p6-2200-2 et pP4-33.
- Figure 5 :: Représentation schématique du plasmide pSK-Kan401 et construction des plasmides pP4-Kan et pP4R-Kan.
- Figure 6 :: Construction et représentation des plasmides pP4-GUS et pSK-GUS.
- Figure 7 :: Représentation schématique du plasmide pKan707 et construction des dérivés du phage M13, pYG64 et pYG65.
- Figure 8 :: Construction et représentation des plasmides pYG69 et pYG70.
- Figure 9 :: Construction et représentation du plasmide pYG72.
- Figure 10 :: Représentation schématique du plasmide pYG404 et construction du plasmide pYG404ΔH.
- Figure 11 :: Construction et représentation du plasmide pYG128.
- Figure 12 :: Construction et représentation des plasmides pYG131 et pYG132.
- Figure 13 :: Construction et représentation des plasmides pP4-15Kan et pP4-60Kan.
- Figure 14 :: Construction et représentation des plasmides pYG129 et pYG130.
- Figure 15 :: Gel de polyacrylamide natif (5 %) coloré selon le protocole décrit dans l'exemple 7.1.2. et démontrant l'expression de l'activité β-glucuronidase dans la souche MW98-8C transformée avec le plasmide pP4-GUS (lignes 1-6 correspondant à 6 clones indépendants). Les résultats obtenus à partir de la souche MW98-8C transformée avec le plasmide pSK-GUS (témoin) sont donnés aux lignes C.
- Figure 16 :: Gel de polyacrylamide-SDS à 8,5 % après coloration au bleu de Coomassie démontrant la sécrétion de la sérum albumine humaine à partir de la souche MW98-8C transformée avec le vecteur pYG132. Pistes a-c, albumine extrait du plasma humain (Sigma) utilisée comme standard et déposée à des concentrations croissantes (0,5, 1,0 et 1,5 µg par piste). Les autres pistes correspondent à 25 µl de surnageant de culture obtenus à partir des milieux décrits dans l'exemple 7.2.1. Glu, glucose ; Glu/EtOH, glucose/éthanol ; Gly, glycerol ; Gly/EtOH, glycerol/éthanol.
- Figure 17 :: Gel de polyacrylamide-SDS à 8,5 % après coloration au bleu de Coomassie démontrant la sécrétion de la sérum albumine humaine à partir de la souche CBS 293.91 transformée avec le vecteur pYG132. Pistes a-c, albumine extrait du plasma humain (Sigma) utilisée comme standard et déposée à des concentrations croissantes (0,5, 1,0 et 1,5 µg par piste). Les autres pistes correspondent à 25 µl de surnageant de culture obtenus à partir des milieux décrits dans l'exemple 7.2.1. Glu, glucose ; Glu/EtOH, glucose/éthanol ; Gly, glycerol ; Gly/EtOH, glycerol/éthanol.
- Figure 18 :: Gel de polyacrylamide-SDS à 8,5 % après coloration au bleu de Coomassie démontrant la sécrétion de la sérum albumine humaine à partir de la souche CBS 293.91 transformée avec le vecteur pYG130. Pistes a-b, albumine extrait du plasma humain (Sigma) utilisée comme standard et déposée à des concentrations croissantes (0,5 et 1,0 µg par piste). Les autres pistes correspondent à 25 µl de surnageant de culture obtenues à partir des milieux décrits dans l'exemple 7.2.1. Glu, glucose; Glu/EtOH, glucose/éthanol.
- Tableau 1 :: Résumé des différentes constructions effectuées à partir du promoteur KlADH4 entier (fragment de 1,2 kb, soit sous forme d'un fragment BglII-BamHI [pP4-Kan, pP4R-Kan, et pP4-GUS], soit sous forme d'un fragment SalI-HindIII [pYG131 et pYG132]).
- Tableau 2 :: Résumé des différentes constructions effectuées à partir du promoteur KlADH4 tronqué (fragment SacI-BamHI de 0,5 kb dont la séquence nucléotidique est donnée à la figure 2 [pP4-15Kan] ; fragment BgIII-SacI de 0,7 kb correspondant aux positions 1-723 de la séquence nucléotidique donnée à la figure 1 [pP4-60Kan] ; fragment SaII-HindIII de 0,67 kb dont la séquence nucléotidique est donnée à la figure 3 [pYG129 et pYG130]).
- Tableau 3 :: Etude de la régulation de l'expression du gène KlADH4. La présence ou absence de l'activité enzymatique ADH IV mise en évidence selon le protocol décrit dans l'exemple 5 est indiquée par les symboles + ou -.

### TECHNIQUES GENERALES DE CLONAGE

Les méthodes classiques de biologie moléculaire telles que la centrifugation d'ADN plasmidique en gradient de chlorure de césium - bromure d'éthidium, la digestion par les enzymes de restriction, l'électrophorèse sur gel, l'électroélution des fragments d'ADN à partir de gels d'agarose, la transformation dans E.coli, etc, sont décrites dans la littérature (Maniatis et al., "Molecular Cloning : a Laboratory Manual", Cold Spring Harbor Laboratory, Cold Spring Harbor, N.Y., 1986 ; Ausubel et al., (eds.), "Current Protocols in Molecular Biology", John Wiley & Sons, New York 1987).

La mutagénèse dirigée in vitro par oligodésoxynucléotides est effectuée selon la méthode développée par Taylor et al. (Nucleic Acids Res. 13 (1985) 8749-8764) en utilisant le kit distribué par Amersham. Le séquençage de nucléotides est réalisé selon la technique des didéoxy décrite par Sanger et al. (Proc. Natl. Acad. Sci. USA, 74 (1977) 5463-5467). L'amplification enzymatique de fragments d'ADN spécifiques est effectuée par réaction de PCR ("Polymerase-catalyzed Chain Reaction") dans les conditions décrites par Mullis et Faloona (Meth. Enzym., 155 (1987) 335-350) et Saiki et al (Science 230 (1985) 1350-1354), en utilisant un "DNA thermal cycler" (Perkin Elmer Cetus) en suivant les recommandations du fabricant.

### EXEMPLES

### Plasmides et souches utilisés dans les exemples.

Les plasmides suivants ont servi aux différentes étapes de clonage, ou lors de la construction de vecteurs d'expression.
- Vecteur de remplacement Lambda-L47 : Loenen et Brammar, Gene 10 (1980) 249-259 ;
- Plasmide pBR322 (Pharmacia, Uppsala, Suède) ;
- Plasmide pTZ19 (Pharmacia, Uppsala, Suède) ;
- Plasmide pSK-Kan401 : Chen et Fukuhara, Gene 69 (1988) 181-192 ;
- Plasmide pBI221 (Clontech Laboratories, Palo Alto, CA, USA) ;
- Plasmide pBC SK +/- (Stratagène, La Jolla, CA, USA) ;
- Plasmide pYG404 (EP 361 991) ;
- Plasmide pKan707 (EP 361 991) ;
- Bactériophage M13mp7 : Messing et al., Proc. Natl. Acad. Sci. USA 74 (1977) 3652.

Les souches suivantes ont été utilisées pour le clonage, la construction et l'utilisation des promoteurs de l'invention.
- K. lactis CBS2359/152 (n° CBS 289.91)
- K. lactis MW98-8C (n° CBS 579.88)
- K. lactis CBS 293.91

### 1/ Isolement du promoteur KlADH4 de K. lactis.

La séquence présentée sur la figure 1 a été obtenue par criblage d'une banque d'ADN génomique total de Kluyveromyces lactis CBS2359/152 au moyen d'une sonde hétérologue provenant du gène ADH2 de S.cerevisiae. Plus précisément, la banque a été obtenue par clonage au site BamHI du vecteur de remplacement Lambda-L47 du produit d'une digestion partielle par l'enzyme Sau3A de l'ADN de K. lactis CBS2359/152 . La sonde utilisée pour l'hybridation est un fragment EcoRV-BamHI de 980 bp comprenant la région codante du gène de structure ADH2 de S.cerevisiae, à l'exception des 70 premières pb (sonde A). Ce fragment est obtenu par digestion enzymatique à partir d'un plasmide nommé pBR322.ADR2.BSa (Williamson et al., Cell 23 (1981) 605-614 ; Russell et al., J. Biol.Chem. 258 (1983) 2674-2682).

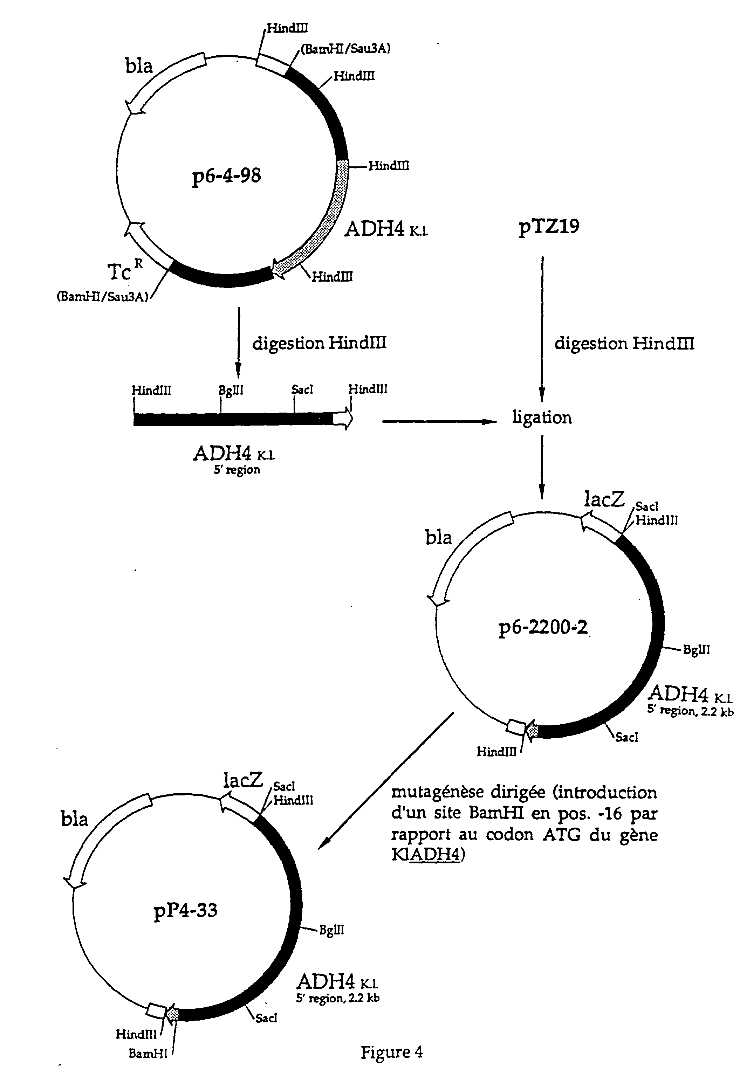

Un fragment de 8 kb environ a ainsi été isolé et sous-cloné au site BamHI du plasmide pBR322 pour générer le plasmide p6-4-98 (figure 4). L'insert BamHI porté par ce plasmide a ensuite été cartographié au moyen d'enzymes de restriction, et la région promotrice du gène KlADH4 a été localisée sur ce fragment par hybridations différentielles en utilisant la sonde A ainsi qu'une deuxième sonde correspondant au fragment BamHI-EcoRV de 1100 pb environ du plasmide pBR322.ADR2.BSa (sonde B).

Dans une seconde étape, le plasmide p6-4-98 a été digéré par l'enzyme HindIII et un fragment de 2,2 kb a été isolé. Ce fragment a ensuite été purifié par des techniques standard et sous-cloné au site HindIII du plasmide pTZ19 pour générer le plasmide p6-2200-2 (figure 4). L'analyse du fragment sous-cloné révèle qu'il contient les 14 premiers codons du gène KlADH4 et la région située en amont de celui-ci, comprenant les éléments de régulation de l'expression.

La partie comprise entre le site BglII et le codon d'initiation de la traduction ATG (fragment de 1,2 kb environ) a été séquencée en utilisant la méthode de terminaison de chaine (Sanger et al., Proc.Nat.Acad.Sci. 74 (1977) 5463). La séquence de ce fragment est présentée sur la figure 1.

### 2/ Construction d'un promoteur KlADH4 portable (BgIII-BamHI)

Un promoteur portable a été préparé par insertion sur le fragment HindIII de 2,2 kb présent sur le plasmide p6-2200-2 d'un site de restriction BamHI en position - 16 par rapport au codon ATG du gène KlADH4.

L'insertion de ce site permet de générer un fragment BglII-BamHI de 1,2 kb comprenant exclusivement la région promotrice KlADH4. Elle permet également d'introduire en aval du promoteur ainsi obtenu tout gène que l'on désire exprimer.

Le site BamHI a été introduit en position -16 par rapport au site d'initiation de la traduction (ATG) du gène KlADH4 par mutagénèse dirigée en utilisant la technique de double amorce (Sambrook, Fritsch, Maniatis, Molecular Cloning Laboratory Manual, Cold Spring Harbor Lab Press, 1989). La séquence des oligodésoxynucléotides synthétiques utilisés pour cette mutagénèse est donnée ci-dessous. Le site BamHI généré est souligné, l'ATG est indiqué en italiques et les astérisques désignent les bases modifiées par rapport à la séquence initiale. SI = Séquence initiale; SM = Séquence modifiée.

5'-CTCCCCCACCAACAACACAACATACAACACACGCAATGTTCAGATT-3'(SI)
** *

5'-CTCCCCCACCAACAACACAGGATCCAACACACGCAATGTTCAGATT-3'(SM)

3'-GAGGGGGTGGTTGTTGTGTCCTAGGTTGTGTGCGTTACAAGTCTAA-5'(SM)

Le plasmide ainsi obtenu est appelé pP4-33 (figure 4).

### 3/ Construction de vecteurs de clonage ou d'expression contenant le promoteur entier.

Le tableau 1 rassemble les constructions décrites dans cet exemple.

### 3.1. Construction d'un vecteur d'expression du gène aph :

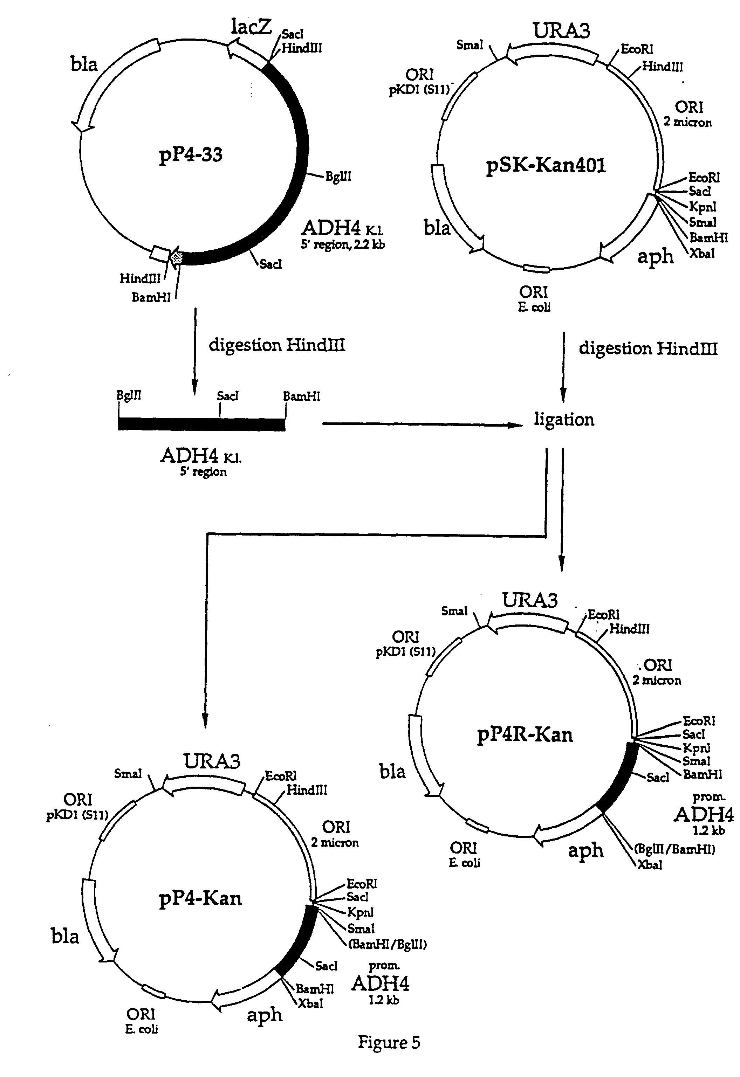

L'ADN du plasmide pP4-33 (Cf exemple 2) a été digéré par BgIII et BamHI pour générer le fragment de 1,2 kb contenant le promoteur KlADH4. Ce fragment a ensuite été introduit au site BamHI du plasmide pSK-Kan401, en amont du gène reporteur aph codant pour l'aminoglycoside-3'-phosphotransférase (I) (Oka et al., J. Mol. Biol. 147 (1981) 217) dépourvu de son propre promoteur, qui, lorsqu'il est exprimé, confère à la levure la résistance à la généticine (G418) (Jimenez et Davies, ref). Après amplification chez E.coli, 2 plasmides recombinants ont été obtenus, différant au niveau de l'orientation de l'insert. Dans le plasmide pP4-Kan le fragment de 1,2 kb est dans la même orientation par rapport au gène aph que celle observée dans le contexte ADH4, alors qu'il se trouve dans l'orientation opposée sur le plasmide pP4R-Kan (voir figure 5).

### 3.2. Construction d'un vecteur d'expression des gènes GUS et aph :

Le plasmide pP4R-Kan a été utilisé pour construire un vecteur d'expression dans lequel 2 gènes hétérologues privés de leurs promoteurs respectifs sont placés de part et d'autre du promoteur KlADH4 de 1,2 kb. Les gènes hétérologues utilisés sont plus spécifiquement :
- le gène aph dont l'expression confère la résistance au G418, et
- le gène de la β-glucuronidase de E.coli (gène GUS), dont l'expression peut être mise en évidence par réaction enzymatique.

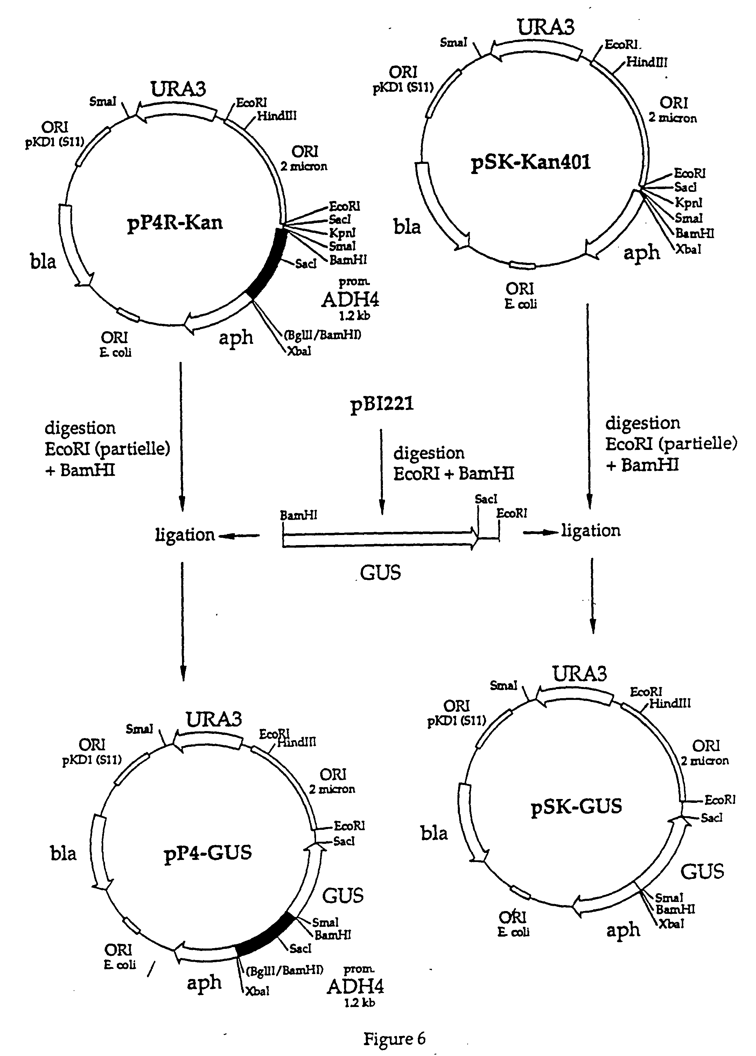

Pour réaliser cette construction, le plasmide pP4R-Kan a été digéré par EcoRI et BamHI. Par ailleurs, à partir du plasmide pBI221, le gène de la β-glucuronidase de E.coli a été isolé sous forme d'un fragment BamHI-EcoRI de 2,1 kb. Ce dernier a ensuite été introduit par ligation dans le plasmide pP4R-Kan linéarisé comme indiqué pour générer le plasmide pP4-GUS (voir figure 6). Parallèlement, un plasmide contrôle a été préparé par insertion du fragment BamHI-EcoRI de 2,1 kb issu du plasmide pBI221 portant le gène GUS dans le plasmide pSK-Kan401 préalablement digéré par BamHI et EcoRI. Le plasmide ainsi obtenu est appelé pSK-GUS (figure 6). Cette construction diffère du plasmide pP4-GUS uniquement par l'absence du promoteur KlADH4 de 1,2 kb.

### 3.3. Construction d'un vecteur d'expression du gène codant pour la sérum albumine humaine (HSA) :

Pour construire différents vecteurs d'expression de la sérum-albumine humaine, un dérivé du plasmide pYG404 (Cf EP 361 991) a été préparé, contenant :
- un réplicon de levure (séquence quasiment entière du plasmide naturel pKD1),
- le gène codant pour la prépro-albumine humaine (HSA) sous contrôle du promoteur du gène LAC4 de K. lactis et suivi du terminateur du gène PGK de S.cerevisiae; le gène de structure codant pour la HSA est précédé par une séquence de 25 nucléotides correspondant à la région directement en amont du gène PGK de S.cerevisiae,
- le gène aph conférant la résistance à la généticine (G418) à la levure, et
- un réplicon et un marqueur de sélection (gène bla conférant la résistance à l'ampicilline) pour E.coli.

Ce plasmide, nommé pYG404ΔH, diffère de pYG404 uniquement par la destruction du site HindIII, localisé dans le gène aph, par mutagénèse dirigée. Cette modification a ensuite permis de substituer le promoteur LAC4 présent dans le plasmide pYG404ΔH sous forme d'un fragment SalI-HindIII par différents variants du promoteur KlADH4 égalements construits comme promoteurs portables sous forme de fragments SalI-HindIII.

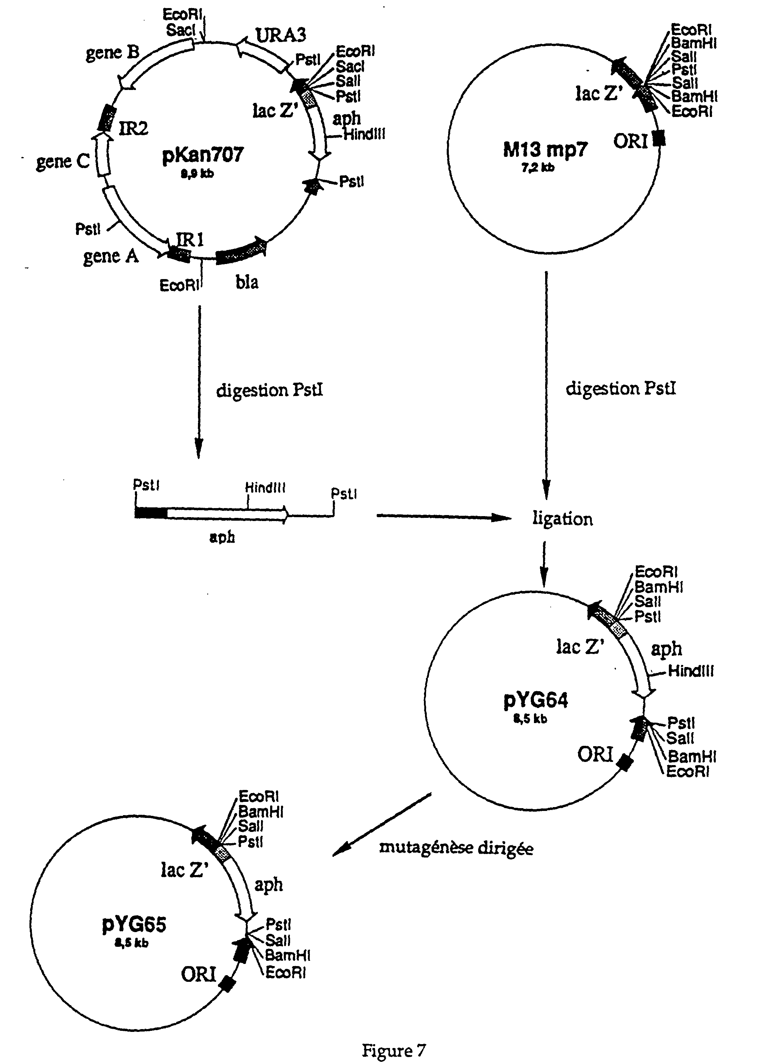

### 3.3.1 Construction du plasmide pYG404ΔH (figures 7-10).

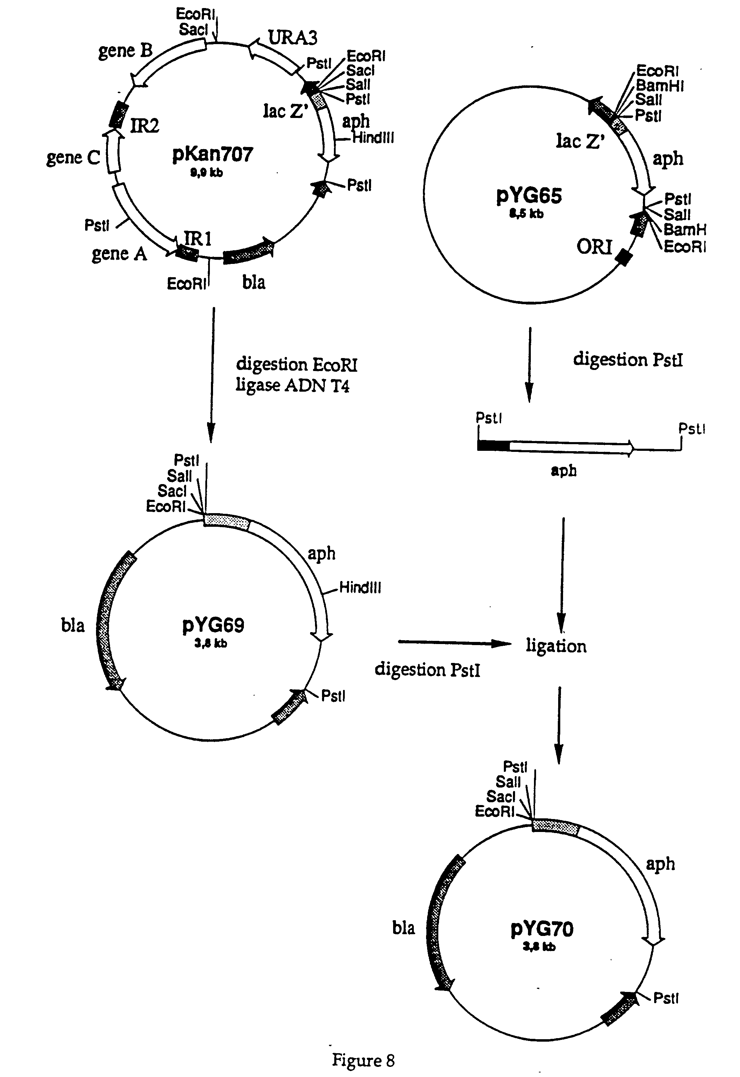

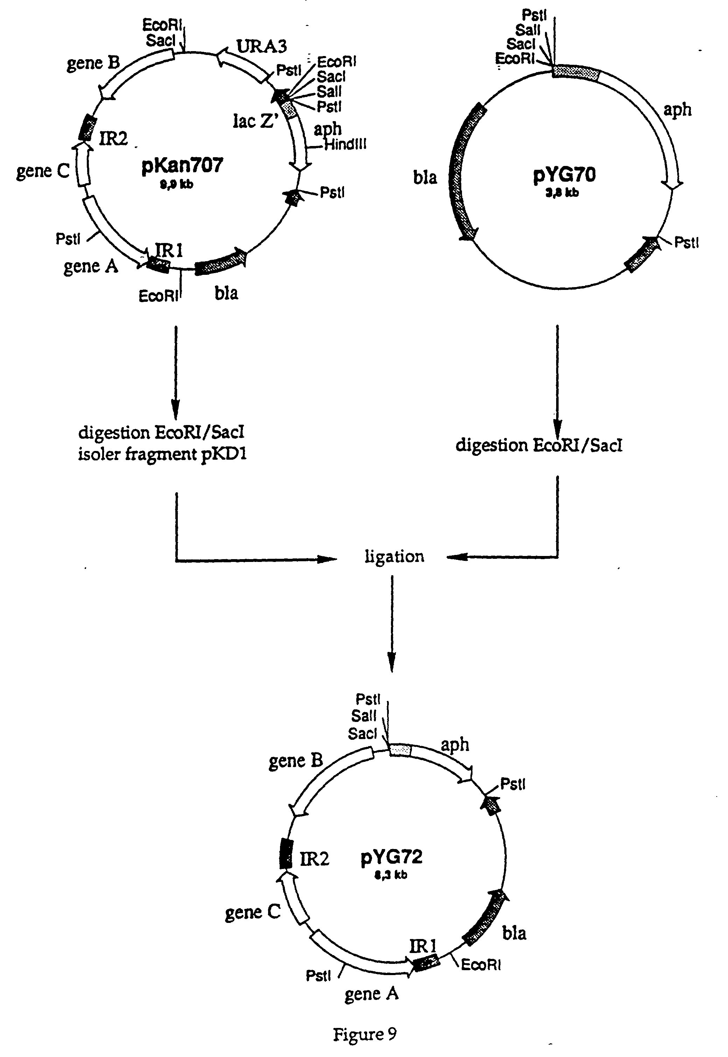

Pour effectuer la délétion du site HindIII dans le vecteur de clonage pYG404, différentes étapes de sous-clonage ont été effectuées, donnant lieu à une construction intermédiaire : pYG72 (figure 9). Ce vecteur correspond au plasmide pKan707 (EP 361 991) dans lequel le fragment SacI contenant le gène URA3 a été éliminé ainsi que le site unique HindIII présent dans le gène aph. Pour effectuer la mutagénèse dirigée sur ce site, le fragment PstI de 1,3 kb portant le gène aph a été sous-cloné à partir du plasmide pKan707 dans le bactériophage M13mp7 pour donner le vecteur pYG64 (figure 7). Le site HindIII a été détruit par mutagénèse dirigée (Cf techniques générales de clonage) en utilisant l'oligodésoxynucléotide suivant : 5'-GAA ATG CAT AAG CTC TTG CCA TTC TCA CCG-3', permettant le remplacement du triplet CTT codant pour la leucine 185 par le triplet CTC. Ce changement ne modifie pas la séquence protéique résultante. Le plasmide obtenu a été appelé pYG65 (figure 7). Pour construire le plasmide pYG72, la partie contenant le réplicon bactérien du vecteur pKan707 a été isolée par digestion avec l'enzyme EcoRI et recircularisation avec la T4 DNA ligase, générant le plasmide intermédiaire pYG69. Le fragment PstI présent dans celui-ci contenant le gène aph a ensuite été remplacé par le fragment équivalent muté provenant du plasmide pYG65. Cette construction a été appelée pYG70 (figure 8). La séquence de pKD1 de 4,7 kb comprise entre les sites EcoRI et SacI a ensuite été introduite dans ce vecteur pour obtenir pYG72 (figure 9).

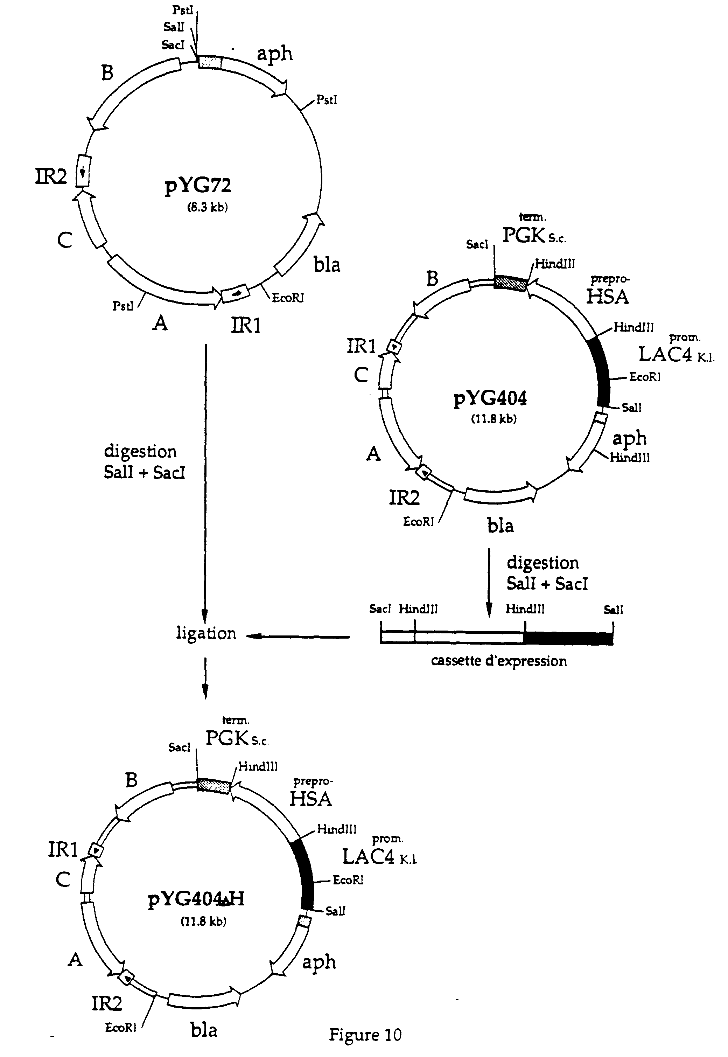

Le vecteur pYG404ΔH a été obtenu en insérant la cassette d'expression provenant du plasmide pYG404 (EP 361 991) sous forme d'un fragment SalI-SacI aux sites correspondants de pYG72 (figure 10).

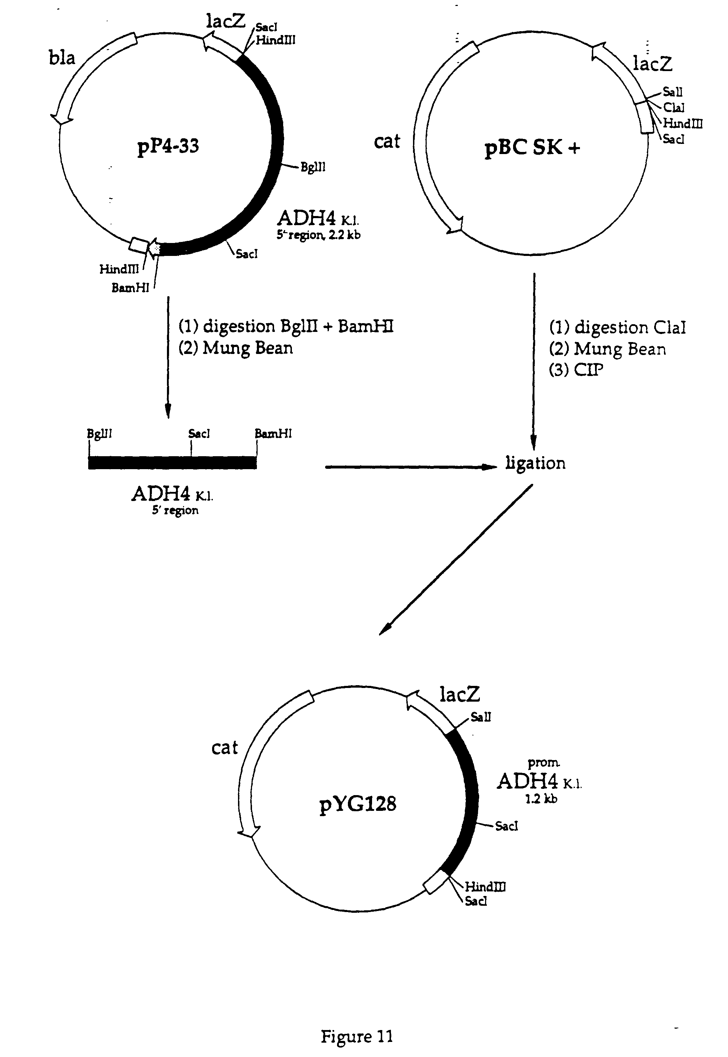

### 3.3.2. Construction d'un promoteur KlADH4 portable [SalI-HindIII] (figure 11) :

Le promoteur KlADH4 porté sur le fragment BglII-BamHI provenant du plasmide pP4-33 (exemple 2) a été modifié de la manière suivante pour l'adapter à l'utilisation dans des vecteurs d'expression dérivés du plasmide pYG404ΔH :

Après digestion du plasmide pP4-33 par les enzymes BglII et BamHI suivi d'un traitement à la nucléase 'Mung Bean' pour rendre les extrémités franches, le fragment de 1,2 kb portant le promoteur KlADH4 a été isolé à partir d'un gel d'agarose et sous-cloné dans le vecteur pBC SK+ (Stratagene, La Jolla, CA, USA) préalablement linéarisé avec l'enzyme ClaI et traité à la nucléase 'Mung Bean' ainsi qu'à la phosphatase alkaline de veau (CIP). Le plasmide obtenu de cette manière (pYG128, figure 11) permet l'isolement du promoteur KlADH4 sous forme d'un fragment SalI-HindIII de 1,2 kb.

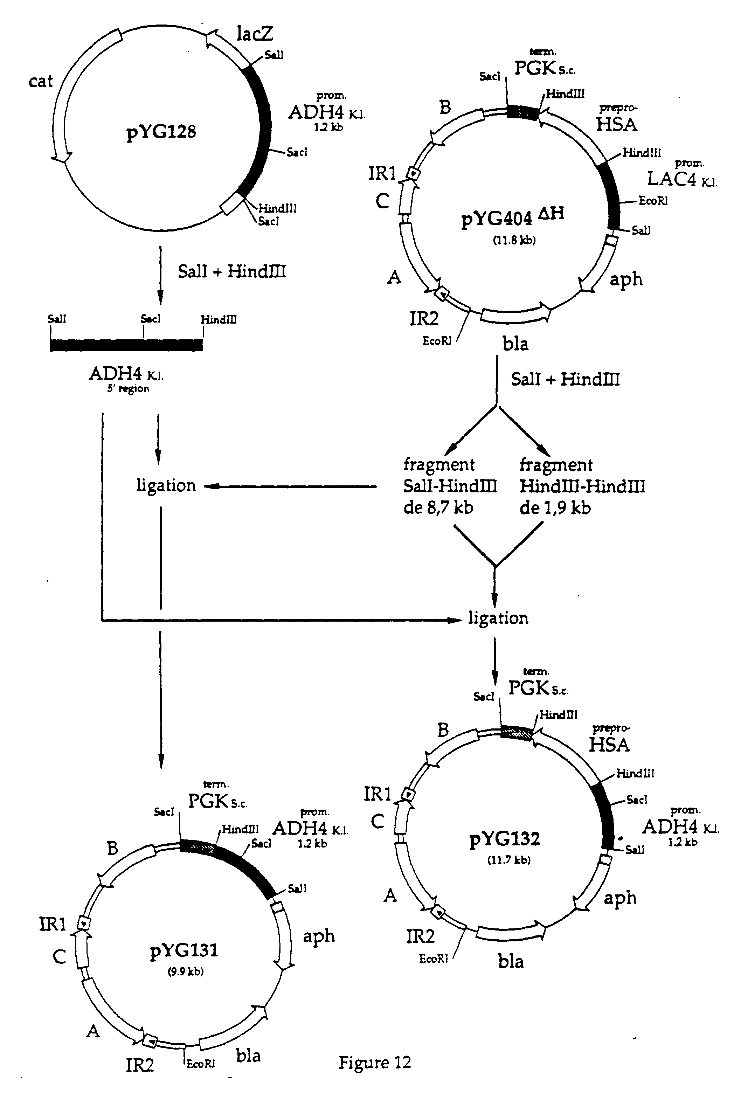

### 3.3.3. Construction des vecteurs pYG131 et pYG132 (figure 12) :

La digestion du vecteur d'expression pYG404ΔH (exemple 3.3.1.) par les enzymes Sali et HindIII permet le remplacement du promoteur LAC4 par le promoteur KlADH4 décrit ci-dessus.

Pour effectuer ce clonage, le fragment SalI-HindIII de 8,7 kb contenant la partie pKD1 et les marqueurs de sélection ainsi que le fragment HindIII-HindIII de 1,9 kb portant le gène codant pour la prépro-HSA ont été isolés à partir du vecteur pYG404ΔH et religués en présence du fragment SalI-HindIII de 1,2 kb provenant du plasmide pYG128 et portant le promoteur KlADH4. Deux plasmides ont été obtenus de cette manière :
- pYG131 (figure 12), correspondant à un vecteur de clonage permettant l'insertion au site HindIII unique de tout gène que l'on désire exprimer sous contrôle du promoteur KlADH4, et
- pYG132 (figure 12), qui est identique au plasmide pYG131 sauf qu'il contient le gène de la prépro-HSA introduit au site HindIII.

### 4/ Construction de vecteurs de clonage ou d'expression contenant des dérivés tronqués du promoteur KlADH4.

Le tableau 2 rassemble les constructions décrites dans cet exemple.

### 4.1. Construction de vecteurs d'expression du gène aph.

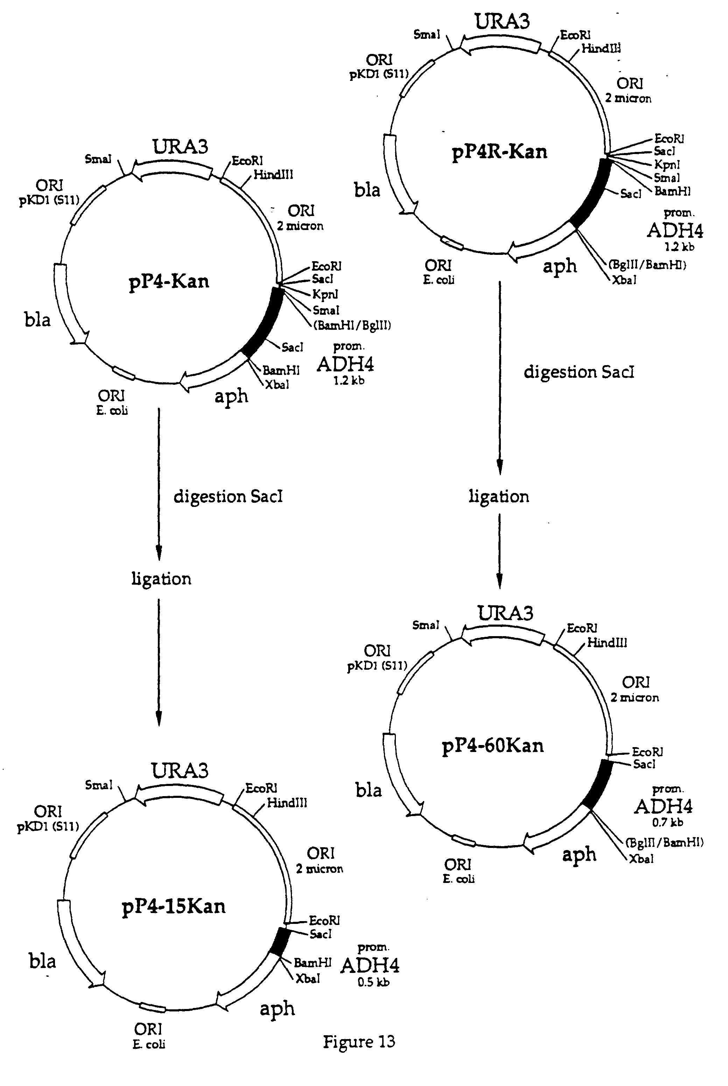

Les plasmides pP4-Kan et pP4R-Kan (exemple 3.1.) ont été utilisés pour préparer des constructions contenant des formes réduites du promoteur. Pour cela, les plasmides pP4-Kan et pP4R-Kan ont été digérés par l'enzyme SacI puis religaturés, selon le schéma décrit sur la figure 13. Cette manipulation a permis :
- d'exciser du plasmide pP4-Kan le fragment SacI de 0,7 kb environ situé du coté opposé au gène aph. Dans le plasmide obtenu (pP4-15Kan) le gène aph est donc sous le contrôle d'un promoteur réduit correspondant au fragment SacI-BamHI de 0,5 kb dont la séquence est donnée sur la figure 2, dans la même orientation que celle observée dans le contexte ADH4.
- d'exciser du plasmide pP4R-Kan le fragment SacI de 0,5 kb environ situé du coté opposé au gène aph. Dans le plasmide obtenu (pP4-60Kan) le gène aph est donc sous le contrôle d'un promoteur réduit correspondant au brin complémentaire du fragment BglII-SacI de 0,7 kb (nucléotides 1 à 723) qui fait partie de la séquence donnée sur la figure 1.

### 4.2. Construction d'un vecteur d'expression du gène de la prépro-HSA :

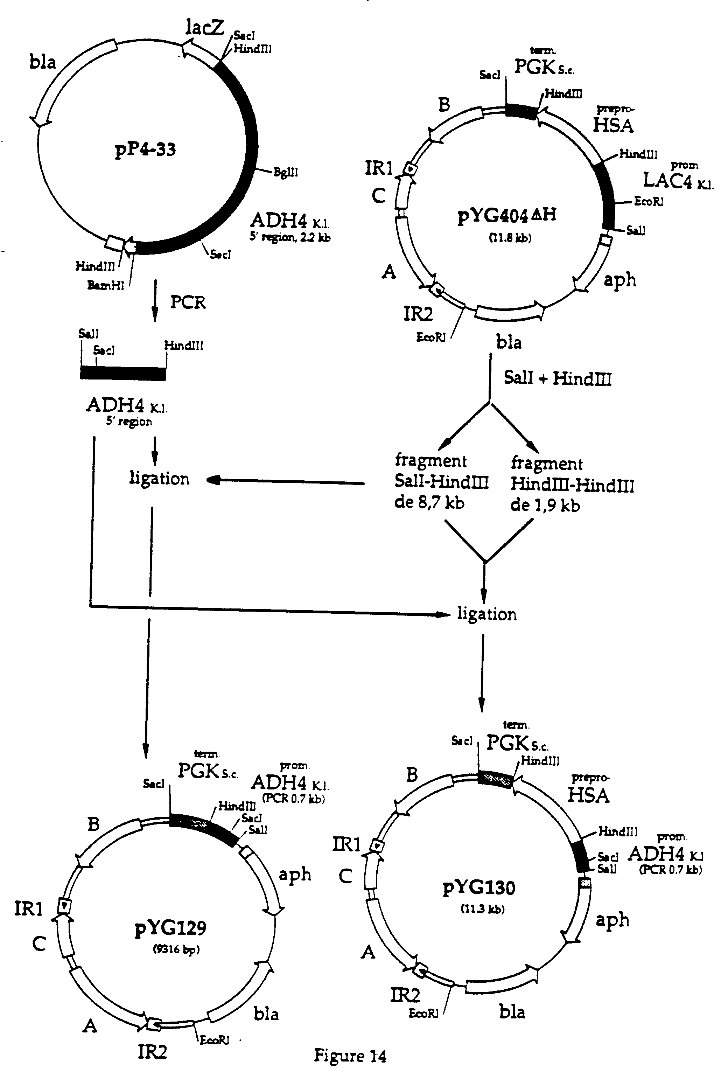

### 4.2.1. Construction d'un promoteur KlADH4 tronqué portable [SalI-HindIII] (figure 14) :

Un dérivé tronqué du fragment BglII-BamHI de 1,2 kb provenant du plasmide pP4-33 et portant le promoteur KlADH4 entier a été obtenu par amplification enzymatique (PCR) d'une partie de ce promoteur, située entre le site BsmAI en position 541 sur la séquence donnée sur la figure 1 et le site BamHI en position -16 par rapport à l'ATG du gène ADH4. Les oligodesoxynucléotides utilisés pour la réaction PCR étaient : - 5'-GGGGTCGACGCGAGACAACACTATTGTGAG-3', introduisant un site SalI (séquence soulignée) juste en amont du site BsmAI précité, et - 5'-GGGAAGCTTTGTGTTGTTGATGGGGGAG -3', remplaçant le site BamHI présent dans la construction pP4-33 par un site HindIII (séquence soulignée).

### 4.2.2. Construction des vecteurs pYG129 et pYG130

Le fragment de 672 bp obtenu par PCR a été digéré par les enzymes SalI et HindIII, purifié à partir d'un gel d'agarose et ligué avec le fragment SalI-HindIII de 8,7 kb contenant la partie pKD1 et les marqueurs de sélection provenant du vecteur 5 pYG404ΔH. Cette ligation a été effectuée en présence du fragment HindIII-HindIII de 1,9 kb portant le gène codant pour la prépro-HSA isolé à partir du même vecteur. Deux plasmides ont été obtenus de cette manière :
- pYG129 (figure 14), correspondant à un vecteur de clonage permettant l'insertion au site HindIII unique de tout gène que l'on désire exprimer sous contrôle du promoteur tronqué KlADH4, et
- pYG130 (figure 14), qui est identique au plasmide pYG129 sauf qu'il contient le gène de la prépro-HSA introduit au site HindIII.

La séquence nucléotidique du fragment SalI-HindIII de 0,7 kb environ correspondant au promoteur KlADH4 tronqué utilisé dans ces deux constructions est 5 donnée sur la figure 3.

### 5/ Etude de la régulation de l'expression du gène KlADH4.

Cette étude a été réalisée avec les souches K. lactis MW98-8C et K. lactis 2359/152.

La souche MW98-8C présente un phénotype Rag2⁻ dû à une mutation (rag2) au niveau du gène codant pour la phosphoglucose isomérase (PGI), qui la rend incapable de produire de l'éthanol sur un milieu glucose. La souche 2359/152 est Rag2⁺.

L'activité du promoteur ADH4 a été déterminée dans différentes conditions de culture, par mise en évidence de l'activité ADH IV en utilisant la technique décrite par Lutstorf et Megnet (Archiv. Biochem. Biophys. 126 (1968) 933). Les résultats obtenus sont rassemblés dans le tableau 3.

Ces résultats indiquent que l'activité du promoteur KlADH4 est spécifiquement induite par l'éthanol et non déreprimée en l'absence du glucose. En effet, nous avons trouvé, d'une manière surprenante, que, contrairement aux promoteurs d'autres alcool deshydrogénases tel que ADH2 de S.cerevisiae ou alcA d'A. nidulans, le promoteur KlADH4 n'est pas reprimé par le glucose (tableau 3). L'éthanol inducteur peut soit être ajouté au milieu de culture soit être produit intracellulairement par fermentation d'une source carbonée tel que le glucose ou le fructose (tableau 3).

Nous avons également montré qu'une souche mutée dans un gène impliqué dans la glycolyse et pour cette raison incapable de produire de l'éthanol à partir du glucose peut être avantageusement utilisée comme hôte pour les vecteurs d'expression de l'invention. En effet, le promoteur KlADH4 est inactif dans un milieu contenant du glucose comme seule source de carbone dans des souches tels que MW98-8C (rag2) dont le gène codant pour la phosphoglucose isomérase est défectif. Dans cette souche, l'activation du promoteur KlADH4 s'effectue par l'addition d'éthanol au milieu de culture.

Par ailleurs, nous avons trouvé, d'une manière surprenante, que dans les souches CBS2359/152 et MW98-8C le promoteur KlADH4 est inactif dans un milieu contenant du glycerol comme seule source de carbone. Ce résultat diffère de ce qui est connu du promoteur du gène de l'alcool deshydrogénase II de S.cerevisiae (ADH2) qui est actif sur différentes sources de carbone non fermentescibles incluant le glycerol.

Ces résultats montrent donc que certaines souches tel que CBS2359/152, même avec un phénotype Rag⁺, peuvent permettre l'obtention d'une expression régulée du promoteur KlADH4 : les cellules cultivées en présence de glycerol comme seule source de carbone ne produisent pas la protéine dont le gène est placé sous contrôle du promoteur KlADH4 (promoteur non-induit) tandis qu'elles peuvent être induite pour la production de cette protéine en ajoutant de l'éthanol au milieu de culture.

### 6/ Transformation de Kluyveromyces.

Différentes techniques permettant l'introduction d'ADN dans la levure peuvent être utilisées.

Avantageusement, les différentes souches de Kluyveromyces utilisées ont été transformées en traitant les cellules entières en présence d'acétate de lithium et de polyéthylène glycol, selon la technique décrite par Ito et al. (J. Bacteriol. 153 (1983) 163-168). La technique de transformation décrite par Durrens et al. (Curr.Genet. 18 (1990) 7) utilisant l'éthylène glycol et le diméthylsulfoxyde a également été utilisée. Il est aussi possible de transformer les levures par électroporation, par exemple selon la méthode décrite par Karube et al. (FEBS Letters 182 (1985) 90).

Un protocole alternatif a encore été décrit en détail dans la demande EP 361 991.

### 7/ Utilisation de vecteurs d'expression pour la production de protéines recombinantes.

### 7.1. Production de protéines bactériennes.

### 7.1.1. Production de l'aminoglycoside 3'-phosphotransférase (I)

Les plasmides d'expression pP4-Kan et pP4R-Kan (exemple 3.1) ainsi que le plasmide de clonage pSK-Kan401 (témoin) ont été utilisés pour transformer la levure K. lactis MW98-8C. Les cellules transformées par pP4-Kan et pP4R-Kan possèdent la résistance au G418 dans un milieu YPD (extrait de levure 10 g/l ; peptone 20 g/l ; glucose 20 g/l) contenant 2 % d'éthanol et des doses de généticine allant de 200 à 400 µg/ml, alors que les cellules transformées par le plasmide pSK-Kan401 sont sensibles à la généticine. Ce résultat indique :
- que le gène aph est exprimé dans les levures transformées par les plasmides pP4-Kan et pP4R-Kan, ce qui démontre que le fragment de 1,2 kb porte bien une activité promotrice fonctionnelle, et,
- que ce fragment porte donc bien une activité promotrice bidirectionnelle, puisque le gène aph est exprimé dans les deux constructions, c'est-à-dire quelle que soit l'orientation du fragment BamHI-BglII de 1,2 kb contenant le promoteur KlADH4.

Les plasmides pP4-15Kan et pP4-60Kan confèrent, après transformation, la résistance à la généticine à la souche MW98-8C cultivée dans un milieu YPD contenant 2 % d'éthanol et des doses de généticine supérieure à 200 µg/ml.

Ce résultat indique clairement que des dérivés actifs du promoteur KlADH4 porté par le fragment 1,2 kb peuvent être obtenus par fragmentation. Il confirme l'activité bidirectionnelle de ce promoteur.

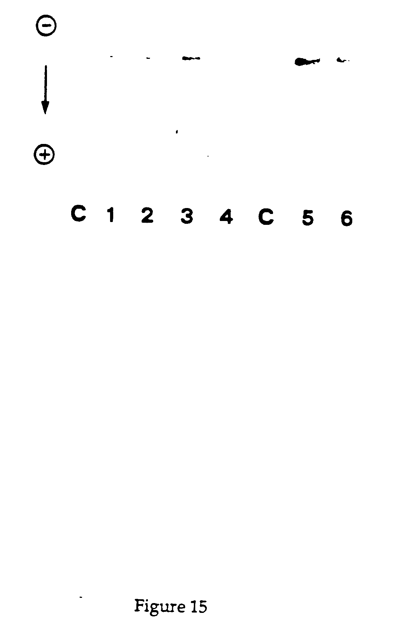

### 7.1.2. Production de la β-glucuronidase (figure 15)

Les plasmides pP4-GUS et pSK-GUS (exemple 3.2) ont été utilisés pour transformer la souche K. lactis MW98-8C (ura3 lysA argA) en sélectionnant les cellules recombinées pour leur prototrophie vis-à-vis de l'uracile sur un milieu synthétique SD ('yeast nitrogen base w/o amino acids' 0,67 %, glucose 2 %) supplémenté avec de l'arginine (20 mg/l) et de la lysine (30 mg/l).

Les cellules recombinées ont été cultivées dans 10 ml de YPD jusqu'à la phase stationnaire. Les cellules ont ensuite été cassées au moyen de billes de verre dans des tubes Eppendorf, centrifugées, et les surnageants analysés par electrophorèse sur minigel (5 % d'acrylamide) en conditions non-dénaturantes. Les gels et tampons utilisés ont été décrits par Williamson et al. (Nature 283 (1980) 214-216).

Des échantillons correspondant à 20-40 µg de protéines ont été séparés par électrophorèse sur gel d'acrylamide (5 %). La migration a été réalisée à 4°C pendant 60 minutes sous un courant de 20 mA.

L'activité β-glucuronidase est mise en évidence en trempant les gels dans une solution colorante contenant 50 mM Na2HPO4, pH 7,0 et 50 µg/ml de bromo-5-chloro-4-indoyl-3 glucuronide (X-Glu) dans 5 mg/ml de diméthylformamide (Jefferson R.A., Plant. Mol. Biol. Report 5 (1987) 387-405).

Les gels ont été maintenus à 37°C dans cette solution jusqu'à apparition des bandes.

Les résultats obtenus sont présentés sur la figure 15. Le gel représenté montre clairement une bande dans les lignes 1 à 6, correspondant aux cellules MW98-8C transformées par le plasmide pP4-GUS, et aucun signal dans les lignes C correspondant aux cellules MW98-8C transformées par le plasmide pSK-GUS, qui ne contient pas de région promotrice de l'invention.

Par ailleurs, les cellules transformées avec les plasmides pP4-GUS et pSK-GUS ont également été cultivées sur un milieu YPD supplémenté par de la généticine (200 mg/l) Les transformants contenant pP4-GUS ont acquis la résistance contre cette antibiotique tandis que la souche contrôle contenant pSK-GUS reste sensible.

Ces résultats confirment l'activité promotrice bidirectionnelle du fragment 1,2 kb. Ils montrent de plus que ce fragment peut être utilisé pour l'expression simultanée de deux gènes hétérologues insérés de part et d'autre du promoteur K1ADH4, dans les 2 orientations opposées.

### 7.2. Production de protéines mammifères

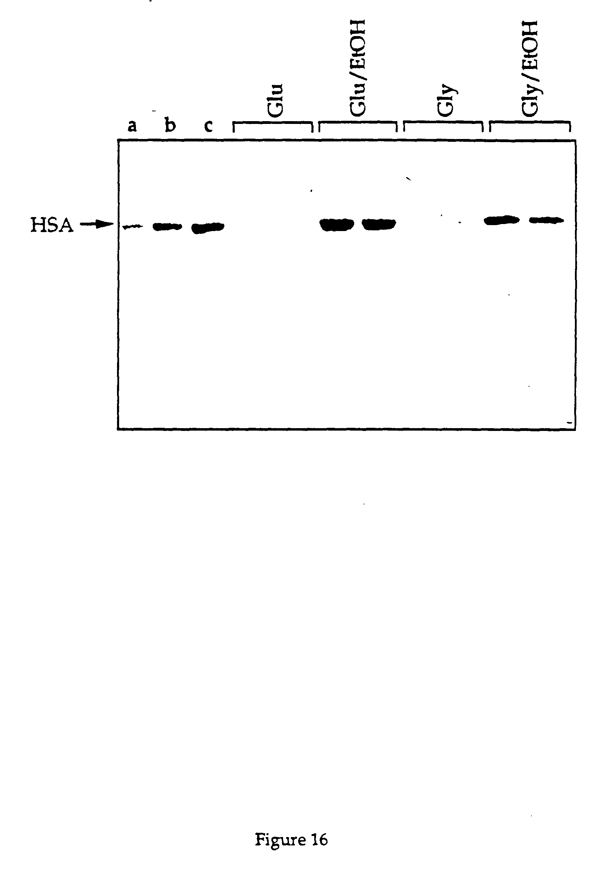

### 7.2.1. Expression du gène de la prépro-HSA sous contrôle du promoteur KlADH4 entier (figures 16-17) :

Le plasmide d'expression pYG132 a été utilisé pour transformer les souches de levures K. lactis MW98-8C (Rag²⁻, incapable de produire de l'éthanol à partir de glucose) et CBS 293.91 (Rag²⁺, métabolisant le glucose pour produire de l'éthanol). Après sélection des cellules recombinantes sur milieu YPD supplémenté par de la généticine (200 mg/l), les transformants ont été précultivés pendant 20 heures environ dans des flacons Erlenmeyer dans un milieu M9EL10 (milieu M9 [Maniatis et al., précité] supplémenté de 10 g/l d'extrait de levure) en présence de glucose (20 g/l) à 28°C sous agitation. Cette préculture a ensuite été utilisée pour inoculer, à une dilution de 10⁻³, des Erlenmeyers de 300 ml contenant 50 ml de milieu M9EL10 en présence de différentes sources de carbone (soit glucose seul [20 g/l], soit glucose [20 g/l] plus éthanol [20 g/l], soit glycerol seul [20 g/l], soit glycerol [20 g/l] plus éthanol [20 g/l]).

Après culture des cellules recombinantes pendant 5 jours à 28°C sous agitation, des échantillons du surnageant de chaque culture, dépourvus de cellules, ont été prélevés et mélangés à un volume équivalent de tampon Laemmli 2X (Laemmli, Nature 227 [1970] 680). Après chauffage à 96° C pendant 10 minutes, les protéines contenues dans un équivalent de 25 µl de surnageant on été séparées (25 mA) sur gel de polyacrylamide SDS 8,5 %. La sécrétion d'albumine a ensuite été révélée par coloration du gel au bleu de Coomassie, et évaluée par densitométrie (densitomètre Shimazu CS930).

La figure 16 montre le résultat obtenu avec la souche MW98-8C/pYG132 pour laquelle on observe une sécrétion d'albumine à haut niveau quand les cellules ont été cultivées en présence d'éthanol ajouté au milieu de culture. Par contre, on ne détecte pas de HSA dans le surnageant quand les cellules ont été cultivées en présence de glucose ou de glycerol.

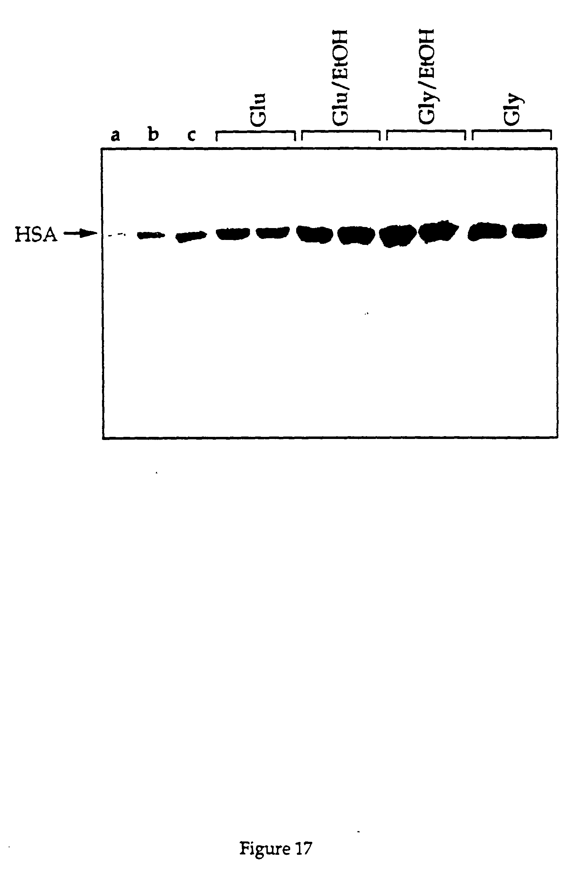

Contrairement aux résultats obtenus avec la souche MW98-8C/pYG132, les transformants CBS293.91/pYG132 sont capables de produire de la HSA dans toutes les conditions de cultures évoquées ci-dessus (figure 17). Néanmoins, la production d'albumine est 2 à 3 fois plus élevée dans une culture contenant de l'éthanol ajouté au milieu par rapport à une culture où l'éthanol est le produit du métabolisme cellulaire.

Ces résultats confirment la capacité des couples hôte/vecteurs décrits dans les exemples précédents à produire à des niveaux élevés, en condition d'induction, une protéine recombinante, qu'elle soit d'origine bactérienne ou mammifère. Par exemple, la production d'albumine en Erlenmeyers dans la souche CBS 293.91/ pYG132 dans un milieu contenant du glycerol ou du glucose en présence d'éthanol (figure 17) a été estimée par densitométrie à 190 - 200 mg/l.

Ces résultats confirment également l'absence de répression du promoteur KlADH4 par le glucose puisque la production d'albumine n'est pas réduite en présence de celui-ci.

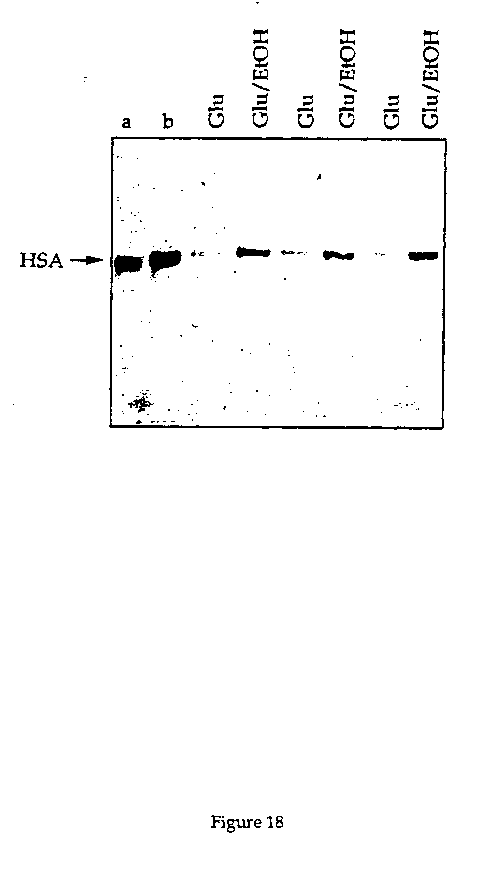

### 7.2.2. Expression du gène de la prépro-HSA sous contrôle du promoteur KlADH4 tronqué (figure 18) :

Le plasmide d'expression pYG130 a été utilisé pour transformer la souche K. lactis CBS 293.91 (Rag2⁺). Après sélection des cellules recombinantes sur milieu YPD supplémenté par de la généticine (200 mg/l), les transformants ont été précultivés et cultivés comme décrit dans l'exemple précédent. La sécrétion d'albumine a été évaluée par électrophorèse d'échantillons de surnageants de culture comme décrit sous 7.2.1. Le résultat obtenu avec le dérivé tronqué du promoteur KlADH4 est montré à la figure 18: le couple hôte/vecteur CBS293.91/pYG130 est clairement capable de produire et sécréter de l'albumine recombinante. Comme dans l'exemple du plasmide pYG132 (promoteur KlADH4 entier) on observe une production d'albumine dans le milieu contenant du glucose comme seule source de carbone, mais à un niveau moins élevé par rapport au promoteur entier. Par contre, la culture des cellules dans un milieu contenant du glucose plus de l'éthanol augmente la production de HSA d'un facteur 2 à 3 (figure 18).

Un échantillon de la souche K. lactis 2359/152 a été déposé le 4 juin 1991 auprès du Centraalbureau voor Schimmelkulturen (CBS) à Baarn aux Pays-Bas dans les conditions du Traité de Budapest, sous le numéro CBS 289.91. La souche K. lactis CBS 293.91 correspond à la souche CBS1065 redéposée le 11 juin 1991 selon les conditions du Traité de Budapest.

**TABLEAU 1**

| **VECTEUR** | **PLASMIDE** **NAVETTE** | **CASSETTE D'EXPRESSION** | |
|---|---|---|---|
| pP4-Kan | pSK-Kan401 | | 1.2 > aph |
| pP4R-Kan | pSK-Kan401 | aph | 1.2 > |
| pP4-GUS | pSK-Kan401 | aph | 1.2 > GUS |
| pYG131 | pYG404D | | 1.2 > NEANT |
| pYG132 | pYG404D | | 1.2 > HSA |

**TABLEAU 2**

| **VECTEUR** | **PLASMIDE** **NAVETTE** | **CASSETTE** **D'EXPRESSION** | |
|---|---|---|---|
| P4-15Kan | pSK-Kan401 | | 0,5 > aph |
| pP4-60Kan | pSK-Kan401 | aph | 0,7 |
| pYG129 | pYG404DH | | 0,67 > NEANT |
| pYG130 | pYG404DH | | 0,67 > HSA |

**TABLEAU 3**

| **Souche\Milieu** | **Glucose** | **Ethanol** | **Glucose+ Ethanol** | **Fructose** | **Glycerol** |
|---|---|---|---|---|---|
| 2359/152 (Rag2+) | + | + | + | + | - |
| MW98-8C (Rag2-) | - | + | + | + | - |

## Revendications

1. Séquence d'ADN consititué[e] de tout ou partie de la séquence présentée à la figure 1 ou de son brin complémentaire, ou d'un dérivé de celles-ci, et possédant une activité de promoteur transcriptionnel.

2. Séquence d'ADN selon la revendication 1 constituté[e] de tout ou partie du fragment SacI-BamHI de 0,5 kb environ présenté sur la figure 2 ou du fragment SalI-HindIII de 0,7 kb environ présenté sur la figure 3.

3. Séquence d'ADN selon la revendication 1 constitué[e] de tout ou partie du fragment BglII-SacI de 0,7 kb environ correspondant au fragment compris entre les nucléotides 1 et 723 du brin complémentaire de la séquence donnée sur la figure 1.

4. ADN recombinant comprenant une séquence d'ADN selon l'une quelconque des revendications 1 à 3 et un ou plusieurs gènes de structure à l'exception du gène KlADH4 de K.lactis).

5. ADN recombinant selon la revendication 4 **caractérisé en ce qu'**il contient également des signaux permettant la sécretion du produit d'expression du ou desdits gènes de structure.

6. ADN recombinant selon les revendications 4 et 5 **caractérisé en ce que** le ou les gènes de structure codent pour des protéines d'intérêt pharmaceutique ou agroalimentaire.

7. ADN recombinant selon la revendication 6 **caractérisé en ce que** le ou les gènes de structure codent pour des protéines choisies parmi les enzymes (tels que notamment la superoxide dismutase, la catalase, les amylases, les lipases, les amidases, la chymosine etc.), les dérivés sanguins (tels que la sérum-albumine, l'alpha- ou la béta-globine, le facteur VIII, le facteur IX, le facteur de von Willebrand, la fibronectine, l'alpha-1 antitrypsine etc.), l'insuline et ses variants, les lymphokines (telles que les interleukines, les interférons, les facteurs de stimulation des colonies (G-CSF, GM-CSF, M-CSF...), le TNF, le TRF etc.), les facteurs de croissance (tels que l'hormone de croissance, l'érythropoiétine, le FGF, l'EGF, le PDGF, le TGF etc.), les apolipoprotéines, des polypeptides antigéniques pour la réalisation de vaccins (hépatite, cytomégalovirus, Eppstein-Barr, herpes etc.), ou encore des fusions de polypeptides telles que notamment des fusions comportant une partie active fusionnée à une partie stabilisatrice (par exemple des fusions entre l'albumine ou des fragments d'albumine et le récepteur ou une partie d'un récepteur de virus (CD4, etc.)).

8. ADN recombinant selon l'une quelconque des revendications 4 à 7 **caractérisé en ce qu'**il fait partie d'un plasmide d'expression, qui peut être à réplication autonome ou intégratif.

9. Cellule recombinée contenant une séquence d'ADN ou un ADN recombinant selon l'une quelconque des revendications précédentes.

10. Cellule recombinée selon la revendication 9 **caractérisée en ce qu'**il s'agit d'une levure.

11. Cellule recombinée selon la revendication 10 **caractérisée en ce qu'**il s'agit d'une levure du genre Kluyveromyces.

12. Utilisation d'une séquence d'ADN selon l'une quelconque des revendications 1 à 8 pour l'expression de gènes recombinés.

13. Utilisation selon la revendication 12 pour l'expression simultanée de gènes recombinés insérés de part et d'autre du promoteur dans les 2 orientations opposées.

14. Utilisation selon l'une des revendications 12 ou 13 pour l'expression de gènes codant pour des protéines d'intérêt pharmaceutique ou agroalimentaire.

15. Procédé de production de protéines recombinantes **caractérisé en ce que** l'on cultive une cellule recombinée selon l'une quelconque des revendications 9 à 11 et on récupère les protéines produites.

16. Procédé selon la revendication 15 pour la production de protéines d'intérêt pharmaceutique ou agroalimentaire.

17. Procédé selon la revendication 16 **caractérisé en ce que** la protéine est préférentiellement la sérum-albumine humaine ou un de ses variants moléculaires.

## Claims

1. DNA sequence consisiting of all or part of the sequence presented in Figure 1 or of its complementary strand, or of a derivative of these sequences, and possessing transcription promoter activity.

2. DNA sequence according to Claim 1, consisting of all or part of the approximately 0.5-kb SacI-BamHI fragment presented in Figure 2 or of the approximately 0.7-kb SalI-HindIII fragment presented in Figure 3.

3. DNA sequence according to Claim 1, consisting of all or part of the approximately 0.7-kb BglII-SacI fragment corresponding to the fragment bounded by nucleotides 1 and 723 of the strand complementary to the sequence given in Figure 1.

4. Recombinant DNA comprising a DNA sequence according to any one of Claims 1 to 3 and one or more structural genes with the exception of the K. lactis KlADH4 gene.

5. Recombinant DNA according to Claim 4, **characterized in that** it also contains signals enabling the expression product of the said structural gene or genes to be secreted.

6. Recombinant DNA according to Claims 4 and 5, **characterized in that** the structural gene or genes code(s) for proteins of pharmaceutical or agri-foodstuffs interest.

7. Recombinant DNA according to Claim 6, **characterized in that** the structural gene or genes code(s) for proteins chosen from enzymes (such as, in particular, superoxide dismutase, catalase, amylases, lipases, amidases, chymosin, and the like), blood derivatives (such as serum albumin, alpha- or beta-globin, factor VIII, factor IX, von Willebrand's factor, fibronectin, alpha₁-antitrypsin, and the like), insulin and its variants, lymphokines (such as interleukins, interferons, colony stimulating factors [G-CSF, GM-CSF, M-CSF, etc.], TNF, TRF, and the like), growth factors (such as growth hormone, erythropoietin, FGF, EGF, PDGF, TGF, and the like), apolipoproteins, antigenic polypeptides for the production of vaccines (hepatitis, cytomegalovirus, Eppstein-Barr, herpes, and the like), or alternatively fusions of polypeptides such as, in particular, fusions containing an active portion fused to a stabilizing portion (e.g. fusions between albumin or albumin fragments and the virus receptor or portion of a virus receptor [CD4, and the like]).

8. Recombinant DNA according to any one of Claims 4 to 7, **characterized in that** it forms part of an expression plasmid, which can be autonomously replicating or integrative.

9. Recombinant cell containing a DNA sequence or a recombinant DNA according to any one of the preceding claims.

10. Recombinant cell according to Claim 9, **characterized in that** it is a yeast.

11. Recombinant cell according to Claim 10, **characterized in that** it is a yeast of the genus Kluyveromyces.

12. Use of a DNA sequence according to any one of Claims 1 to 8 for the expression of recombinant genes.

13. Use according to Claim 12, for the simultaneous expression of recombinant genes, inserted on each side of the promoter, in the 2 opposite orientations.

14. Use according to one of Claims 12 and 13, for the expression of genes coding for proteins of pharmaceutical or agri-foodstuffs interest.

15. Method for the production of recombinant proteins, **characterized in that** a recombinant cell according to any one of Claims 9 to 11 is cultured and the proteins produced are recovered.

16. Process according to Claim 15, for the production of proteins of pharmaceutical or agri-foodstuffs interest.

17. Process according to Claim 16, **characterized in that** the protein is preferably human serum albumin or one of its molecular variants.

## Patentansprüche

1. DNA-Sequenz bestehend aus der Gesamtheit oder einem Teil der in Figur 1 dargestellten Sequenz oder deren Komplementärstrang oder einem Derivat davon und im Besitz einer Aktivität eines Transkriptionspromotors.

2. DNA-Sequenz nach Anspruch 1, bestehend aus der Gesamtheit oder einem Teil des etwa 0,5 kb großen SacI-BamHI-Fragments, das in Figur 2 dargestellt ist, oder des etwa 0,7 kb großen SalI-HindIII-Fragments, das in Figur 3 dargestellt ist.

3. DNA-Sequenz nach Anspruch 1, bestehend aus der Gesamtheit oder einem Teil des etwa 0,7 kb großen BglII-SacI-Fragments, das dem Fragment zwischen den Nukleotiden 1 und 723 des Komplementärstrangs der in Figur 1 angegeben Sequenz entspricht.

4. Rekombinante DNA, umfassend eine DNA-Sequenz nach einem der Ansprüche 1 bis 3, und ein oder mehrere Strukturgene, ausgenommen das KlADH4-Gen von K. lactis.

5. Rekombinante DNA nach Anspruch 4, **dadurch gekennzeichnet, dass** sie auch Signale enthält, die die Sekretion des Expressionsprodukts des oder der Strukturgene erlauben.

6. Rekombinante DNA nach den Ansprüchen 4 und 5, **dadurch gekennzeichnet, dass** das oder die Strukturgen(e) Proteine von pharmazeutischem oder agroalimentärem Interesse codiert/codieren.

7. Rekombinante DNA nach Anspruch 6, **dadurch gekennzeichnet, dass** das oder die Strukturgen(e) Proteine, ausgewählt aus Enzymen (wie insbesondere Superoxiddismutase, Katalase, Amylasen, Lipasen, Amidasen, Chymosin etc.), Blutderivaten (wie Serum-Albumin, alphaoder beta-Globin, Faktor VIII, Faktor IX, von-Willebrand-Faktor, Fibronectin, alpha-1-Antitrypsin etc.), Insulin und dessen Varianten, Lymphokinen (wie Interleukine, Interferone, koloniestimulierende Faktoren (G-CSF, GM-CSF, M-CSF..., TNF, TRF etc.), Wachstumsfaktoren (wie Wachstumshormon, Erythropoietin, FGF, EGF, PDGF, TGF etc.), Apolipoproteine, antigene Polypeptide zur Herstellung von Impfstoffen (Hepatitis, Cytomegalievirus, Eppstein-Barr, Herpes etc.) oder auch Fusionen von Polypeptiden, wie insbesondere Fusionen, umfassend einen aktiven Teil, fusioniert an einen stabilisierenden Teil (beispielsweise Fusionen zwischen Albumin oder Albuminfragmenten und dem Rezeptor oder einem Teil eines Virusrezeptors (CD4 etc.)) codiert/codieren.

8. Rekombinante DNA nach einem der Ansprüche 4 bis 7, **dadurch gekennzeichnet, dass** sie Teil eines Expressionsplasmids ist, das der autonomen oder integrativen Replikation dient.

9. Rekombinante Zelle, enthaltend eine DNA-Sequenz oder eine rekombinante DNA nach einem der vorstehenden Ansprüche.

10. Rekombinante Zelle nach Anspruch 9, **dadurch gekennzeichnet, dass** es sich um eine Hefe handelt.

11. Rekombinante Zelle nach Anspruch 10, **dadurch gekennzeichnet, dass** es sich um eine Hefe der Gattung Kluyveromyces handelt.

12. Verwendung einer DNA-Sequenz nach einem der Ansprüche 1 bis 8 zur Expression von rekombinanten Genen.

13. Verwendung nach Anspruch 12 zur gleichzeitigen Expression von rekombinanten Genen, die beiderseits des Promotors in den zwei entgegengesetzten Orientierungen insertiert sind.

14. Verwendung nach einem der Ansprüche 12 oder 13 zur Expression von Genen, die Proteine von pharmazeutischem oder agroalimentärem Interesse codieren.

15. Verfahren zur Produktion von rekombinanten Proteinen, **dadurch gekennzeichnet, dass** man eine rekombinante Zelle nach einem der Ansprüche 9 bis 11 züchtet und die gebildeten Proteine isoliert.

16. Verfahren nach Anspruch 15 zur Produktion von Proteinen von pharmazeutischem oder agroalimentärem Interesse.

17. Verfahren nach Anspruch 16, **dadurch gekennzeichnet, dass** das Protein bevorzugt Humanserumalbumin oder eine seiner molekularen Varianten ist.
